# FASCICULE DE BREVET EUROPEEN

(11) **EP 2 747 738 B1**
(45) Date de publication et mention de la délivrance du brevet: **06.12.2017**
(21) Numéro de dépôt: 12758551.1
(22) Date de dépôt: 07.08.2012
(51) Int. Cl.: A61Q 1/10, A61K 8/81, A61K 8/86, A61K 8/02

(54) **MASCARA SOUS FORME DE POUDRE**
PULVER MASCARA
MASCARA IN POWDER FORM

(30) Priorité: 26.08.2011 FR 1157552
(43) Date de publication de la demande: 02.07.2014
(73) Titulaire: LvmH Recherche, 45800 Saint-Jean De Braye (FR)
(72) Inventeur: TRANCHANT, Jean-François, R., F-45760 Marigny Les Usages (FR); GOMBART, Emilie, J., F-45100 Orleans (FR)
(74) Mandataire: Mena, Sandra
(86) Numéro de dépôt international: PCT/FR2012/051857
(87) Numéro de publication internationale: WO 2013/030485

(56) Documents cités:
- EP-A1- 1 466 580
- EP-A2- 1 955 610
- FR-A1- 2 824 267
- FR-A1- 2 905 068
- FR-A1- 2 923 381
- DATABASE GNPD [Online] Mintel; avril 2002 (2002-04), "Cake Mascara", XP002674408, Database accession no. 93035

## Description

L'objet de l'invention porte sur une composition de mascara, qui doit être chauffée pour être appliquée sur les fibres kératiniques.

Les mascaras se présentent généralement sous la forme d'une pâte en émulsion dans laquelle des huiles et des cires sont dispersées dans une phase aqueuse. Il existe également des mascaras sous forme d'une émulsion inverse, dans laquelle l'eau est dispersée dans la phase grasse, ou encore des mascaras pains solides obtenus par mélange de poudres dans un corps gras préalablement chauffé et fondu, lequel mélange est ensuite coulé à chaud dans une coupelle avant de refroidir. Ces mascaras, qui comprennent généralement des polymères filmogènes et des pigments se présentent habituellement sous forme liquide ou pâteuse et sont appliquées sur les cils à froid à l'aide d'une brosse.

On a récemment vu apparaître des mascaras qui sont chauffés avant ou pendant leur application sur les cils. Ces compositions, dénommées dans la présente demande « mascaras appliqués à chaud », sont habituellement conditionnées sous la forme de kits comprenant un réservoir, un applicateur et un dispositif de chauffage. Il existe de nombreuses variantes de ces kits selon que le dispositif de chauffage est situé au niveau du réservoir ou dans la brosse. Le moyen de chauffage peut aussi être dissocié du conditionnement de la composition de mascara.

Les compositions de l'art antérieur sont des mascaras sous forme d'une masse solide ou pâteuse à température ambiante qui sont conditionnés dans des réservoirs dans lesquels on prélève une partie à l'aide d'un dispositif comprenant des moyens de chauffage (voir par exemple la demande FR 2853504).

Les compositions comprennent des composés qui présentent un pic de fusion adapté à une telle utilisation et généralement comprennent une phase aqueuse continue.

La demande FR 2903600 divulgue un mascara comprenant une phase aqueuse et un polymère thermogélifiant.

La demande FR 2891820 divulgue une composition comprenant une phase aqueuse et une phase grasse comprenant elle-même des polyorganosiloxanes.

Les mascaras appliqués à chaud subissent à chaque application sur les cils, un cycle de chauffage et de refroidissement à l'issue duquel la composition reprend sa consistance initiale.

La répétition de ces cycles découlant d'une utilisation régulière du mascara, habituellement quotidienne, entraîne généralement une altération des propriétés de la composition lorsqu'elle contient un ingrédient qui se dégrade à la chaleur, ou lorsqu'elle n'est pas stable thermiquement. Après un nombre limité de cycles, la composition devient plus visqueuse, jusqu'à prendre parfois en masse ; la composition peut aussi perdre ses propriétés de recouvrement et d'adhésion sur les fibres kératiniques.

Les compositions de mascara comprenant une phase aqueuse comme les mascaras sous forme d'émulsion huile-dans-eau, sont particulièrement sensibles à ces cycles de chauffage-refroidissement répétés. En effet, l'évaporation progressive de l'eau du mascara au fur et à mesure des prélèvements, sous l'influence de la chaleur dégagée par l'instrument de chauffage, peut en altérer la texture et la stabilité.

L'invention a pour objectif de proposer une nouvelle forme de mascara appliqué à chaud, qui vise à pallier les inconvénients des mascaras appliqués à chaud de l'art antérieur dont la composition s'altère progressivement au fur et à mesure de leur utilisation.

Le but de l'invention est également de disposer d'une composition de maquillage des fibres kératiniques qui présente de bonnes propriétés cosmétiques, en particulier un dépôt homogène, une adhésion satisfaisante, une bonne tenue dans le temps et un démaquillage aisé.

Le mascara de l'invention permet notamment d'éviter d'avoir à soumettre la composition à une répétition de cycles de chauffage-refroidissement.

Le mascara de l'invention présente l'avantage supplémentaire de pouvoir être prélevé facilement avant l'étape de chauffage, ce qui permet d'adapter la quantité souhaitée, par exemple pour moduler l'effet que l'utilisateur souhaite obtenir. On épargne ainsi au reste de la composition d'avoir à subir un cycle chauffage-refroidissement, ce qui améliore significativement la stabilité du mascara et la durée d'utilisation du produit. Le mascara de l'invention permet ainsi au formulateur de ne pas être limité dans ses choix d'ingrédients à des composés qui dont la structure est sensible à la chaleur.

Un premier objet de l'invention vise ainsi un mascara destiné à être chauffé pour être appliqué sur les fibres kératiniques, en particulier les cils, se présentant sous forme d'une poudre.

Par « mascara », on définit une composition de maquillage destinée à être appliquée sur des fibres kératiniques. Le mascara est plus particulièrement destiné au maquillage ou au traitement cosmétique des fibres kératiniques, telles que les fibres kératiniques humaines (cils, sourcils, cheveux) et les faux-cils.

Il peut s'agir d'une base de maquillage (ou « base-coat »), d'une composition à appliquer sur une base (« top-coat »), ou bien encore d'une composition de traitement cosmétique des fibres kératiniques.

Le mascara de l'invention est avantageusement anhydre.

Le mascara est anhydre en ce sens que de l'eau n'a pas été ajoutée lors de sa fabrication. Il peut néanmoins subsister dans le mascara de l'eau à l'état de traces, notamment moins de 5%, de préférence moins de 3% en poids par rapport au poids de la composition.

Le mascara anhydre de l'invention comprend ainsi une teneur en eau inférieure à 5%, de préférence inférieure à 3% en poids par rapport au poids de la composition.

La taille des particules constituant la poudre est adaptée de façon à permettre sont prélèvement et la charge de l'applicateur chauffant dans une quantité suffisante.

Avantageusement, le mascara est une poudre constituée de particules dont le diamètre moyen est compris 20 µm à 1 mm. La granulométrie des particules est déterminée par des méthodes usuelles telles que le tamisage ou la granulométrie laser.

Selon un mode de mise en oeuvre, les particules de poudre comprennent une seule phase continue qui peut être lipophile ou hydrophile. Dans ce cas, les particules de la poudre ne sont pas des microcapsules, au sens où elles ne comprennent pas deux phases continues, par exemple un coeur liquide dans une coque solide. De telles microcapsules sont notamment décrites dans la demande WO 2006/057438.

Les particules de la poudre sont solides à température ambiante (25°C), et le mascara possède une température de fusion comprise entre 35 et 70°C, de préférence encore allant de 40 à 50°C, de préférence allant de 40 à 45°C. La température de fusion du mascara peut être mesurée par analyse thermique différentielle et correspond à la température du sommet du pic de fusion.

Les particules de poudre constituant le mascara contiennent au moins deux polymères tels que décrits dans les revendications de préférence filmogènes.

Dans la présente invention, on entend par « polymère filmogène », un polymère apte à former un film continu sur un support, à lui seul ou en présence d'un agent adjuvant tel qu'un plastifiant. Le support est de préférence constitué de fibres dont la forme et les dimensions sont celles de fibres kératiniques.

Dans le texte, le mot polymère peut désigner un homopolymère ou un copolymère. Par « copolymère », on entend un polymère comprenant au moins deux monomères ou deux blocs différents, pouvant être de la même famille chimique mais de structure différente. Les polymères filmogènes sont utilisés en mélange, dans des proportions variables, afin d'ajuster la température de fusion de la poudre de mascara.

La quantité totale de polymère filmogène va de préférence de 1 à 95 % en poids sec dudit polymère filmogène par rapport au poids total du mascara. Le mascara comprend au moins un premier polymère, de préférence filmogène, dont la température de fusion est comprise entre 35°C et 70°C, et au moins un deuxième polymère, de préférence filmogène, dont la température de fusion est comprise entre 80°C et 150°C. Selon un mode de réalisation, les deux polymères sont hydrophiles. Selon un autre mode de réalisation les deux polymères sont lipophiles.

La proportion massique entre le premier polymère et le deuxième polymère est avantageusement comprise entre 1 et 20, de préférence entre 3 et 16, de préférence encore entre 4 et 10.

La quantité totale du premier polymère et du deuxième polymère, lorsqu'il est présent, est comprise entre 40 et 95% en poids du poids total du mascara, et mieux de 70 % à 85 % en poids.

Le premier polymère représente de préférence de 55 à 80% en poids, de préférence encore de 60 à 75% en poids, du poids du mascara. Le deuxième polymère représente de préférence de 1 à 20% en poids, de préférence encore de 5 à 15% en poids, du poids total du mascara.

Le premier polymère est choisi parmi les polyalkylène glycols, de préférence de poids moléculaire compris entre 1000 et 3000 g/mol, et les copolymères de vinylpyrrolidone et d'alcène, de préférence de poids moléculaire compris entre 15 000 et 20 000 g/mol.

Le deuxième polymère est alors choisi parmi les polyvinylpyrrolidones, de préférence de poids moléculaire compris entre 10 000 et 100 000 g/mol et les polybutènes, de préférence de poids moléculaire compris entre 300 et 2500 g/mol.

Selon un premier mode de mise en oeuvre préféré de l'invention, la composition est lipophile et contient au moins deux polymères, de préférence filmogènes, le premier polymère étant choisi parmi les copolymères de la vinylpyrrolidone (VP) et d'alcène, de préférence parmi les copolymères VP/eicosène, VP/hexadécène, VP/triacontène, VP/styrène, de préférence de poids moléculaire compris entre 15 000 et 20 000 g/mol, et le deuxième polymère étant choisi parmi les polybutènes ayant de préférence un poids moléculaire moyen avantageusement compris entre 300 et 2500 g/mol.

Selon un deuxième mode de mise en oeuvre préféré de l'invention, la composition est hydrophile et contient deux polymères, de préférence filmogènes, le premier polymère étant choisi parmi les polyalkylène glycols, par exemple les polyéthylènes glycols de préférence de poids moléculaire moyen compris entre 1000 et 3000 g/mol, et le deuxième polymère étant choisi parmi les polyvinylpyrrolidones (PVP), de préférence les polyvinylpyrrolidones de poids moléculaire moyen compris entre 10 000 et 100 000 g/mol, avantageusement compris entre 40 000 et 70 000 g/mol.

Le mascara de l'invention peut en outre comprendre au moins un agent auxiliaire favorisant la formation d'un film avec le polymère filmogène, ledit agent pouvant être choisi parmi les composés connus de l'homme du métier, notamment les agents plastifiants et les agents de coalescence.

Outre le polymère filmogène, le mascara de l'invention comprend une phase grasse continue anhydre dans laquelle le polymère filmogène est avantageusement solubilisé ou dispersé de façon homogène. Cette phase grasse anhydre est avantageusement solide à température ambiante (25°C) et pression atmosphérique (760 mm Hg, soit 105 Pa). Elle peut comprendre au moins une cire choisie parmi des cires naturelles ou synthétiques, solides à une température d'environ 30°C. L'ajout de ladite cire dans la composition vise à épaissir le film et donner une texture plus visqueuse au mascara lors de son application lorsqu'il a été fondu.

Parmi les cires utilisables dans le cadre de l'invention, on peut mentionner
- des cires dites « polaires », telles que par exemple la cire d'abeille, la cire de lanoline; la cire de son de riz, la cire de Carnauba, la cire de Candellila, la cire d'Ouricurry, la cire du Japon, la cire de Berry, la cire de Sumac, la cire de Montan, les cires obtenues par hydrogénation d'huiles animales ou végétales ayant des chaînes grasses, linéaires ou ramifiées, telles que l'huile de jojoba, l'huile de tournesol, l'huile de ricin, l'huile de coprah, l'huile de lanoline, l'huile d'olive estérifiée avec l'alcool stéarylique, l'huile de ricin estérifiée avec l'alcool cétylique,
- des cires dites « apolaires » telles que les cires microcristallines, les paraffines, l'ozokérite, les cires de polyéthylène, les cires de silicone et les cires fluorées, et
- leurs mélanges.

Avantageusement, le mascara comprend de 1 à 25 % en poids, de préférence de 5% à 10 % en poids, d'au moins une cire par rapport au poids total de la composition de mascara.

Selon un mode de mise en oeuvre particulièrement préféré, la composition est anhydre, comprend au moins un polymère filmogène, de préférence encore au moins deux polymères filmogènes, et au moins une cire, la cire représentant de préférence moins de 15% en poids du poids du mascara.

La composition selon l'invention peut également comprendre au moins une matière colorante, avantageusement choisie parmi les pigments, les colorants liposolubles et les nacres. Les particules constituant ces matières colorantes sont de préférence d'un diamètre ne dépassant pas 200 µm, ne dépassant de préférence pas un diamètre de 150 µm.

Les pigments peuvent être blancs ou colorés, minéraux et/ou organiques, enrobés ou non. On cite, parmi les pigments utilisables dans le mascara de l'invention, le dioxyde de titane, éventuellement traité en surface, les oxydes de zirconium, de zinc ou de cérium, ainsi que les oxydes de fer ou de chrome, le violet de manganèse, le bleu outremer, l'hydrate de chrome et le bleu ferrique, le noir de carbone, et les laques à de colorant, notamment les laques de baryum, strontium, calcium ou aluminium.

Les nacres peuvent être choisies parmi les pigments nacrés blancs tels que le mica recouvert de titane ou d'oxychlorure de bismuth, les pigments nacrés colorés tels que le mica titane avec des oxydes de fer, le mica titane avec notamment du bleu ferrique ou de l'oxyde de chrome, le mica titane avec un pigment organique du type précité ainsi que les pigments nacrés à base d'oxychlorure de bismuth.

Le mascara comprend avantageusement de 5% à 30 % en poids, de préférence de 10 à 25% en poids de matière colorante par rapport au poids total du mascara.

Selon un mode de mise en oeuvre préférée, on utilise des pigments dispersés dans une base telle qu'une cire, par exemple des particules d'oxyde de fer dispersées dans une cire.

Le mascara de l'invention comprend de préférence moins de 5% en poids, de préférence moins de 3% en poids, d'un composé liquide à température ambiante, comme par exemple une huile ou un solvant volatile tel que l'isododécane, ou une cyclométhicone comme la cyclopentasiloxane.

La composition de l'invention peut également comprendre tout additif usuellement utilisé en cosmétique tels qu'une charge solide, des antioxydants, des conservateurs, des parfums, des agent actifs cosmétiques destinés au traitement des fibres sur lesquelles est appliquée la composition, comme par exemple des émollients, des hydratants, des vitamines, des filtres solaires, et leurs mélanges.

Un deuxième objet de l'invention vise un procédé de fabrication de la composition selon l'invention comprenant :
- une étape de mélange à chaud des composants du mascara, de façon à former une phase liquide,
- une étape de refroidissement,
- puis une étape visant à diviser la masse préparée précédemment de façon à obtenir une poudre formée de particules solides.

L'étape de division peut être réalisée par broyage de la phase liquide qui a été solidifiée au préalable par refroidissement, ou bien encore par atomisation de la phase liquide, par exemple à l'aide d'un lit d'air fluidisé, ou encore par tout autre procédé permettant d'obtenir des particules solides à partir d'une phase liquide chauffée.

Il est ainsi possible d'optimiser la granulométrie des particules de poudre constituant le mascara de l'invention en adaptant la formule ou en choisissant avec soin les conditions de l'étape de division.La granulométrie est ainsi adaptée pour permettre à la fois d'obtenir un prélèvement facile et reproductible, et également une fusion rapide et complète de la composition pulvérulente au moment de chauffage en vue d'une application sur les fibres kératiniques.

Un troisième objet de l'invention concerne un kit de maquillage comprenant un mascara tel que défini précédemment conditionné dans un réservoir, ainsi que des moyens de prélèvement, d'application et de chauffage dudit mascara.

Le mascara de l'invention est conditionné sous forme non compactée, les particules solides n'étant ainsi pas agrégées ou liées par un quelconque procédé tel que le compactage.

On peut ainsi parler avantageusement de « poudre libre » pour qualifier ce type de composition pulvérulente. Dans ce cas, le mascara est conditionné par écoulement de la poudre dans le réservoir.

Le mascara sous forme de poudre est avantageusement conditionné dans un réservoir, par exemple un flacon, un godet ou un pot.

Le kit peut comprendre un ensemble dans lequel le moyen de conditionnement et le moyen d'application du mascara de l'invention sont solidaires. Le réservoir peut être physiquement séparé du dispositif d'application.

Le moyen de chauffage peut être séparé de l'ensemble de conditionnement et d'application, ou intégré à celui-ci.

L'outil d'application peut également remplir la fonction de prélèvement.

Selon une première variante, le moyen de prélèvement est couplé ou intégré au réservoir.

Le réservoir et/ou le moyen de prélèvement peuvent également comprendre des moyens de distribution d'une quantité de poudre de mascara de l'invention, de façon à ne prélever que la quantité nécessaire à l'application sur les fibres de kératine. Ces moyens de distribution peuvent avantageusement maintenir hermétiquement la masse de poudre dans le réservoir, de façon à la protéger de l'humidité ou de la température.

Selon une deuxième variante, le moyen de prélèvement est couplé ou intégré au moyen d'application du mascara.

Selon une troisième variante, le moyen de prélèvement est physiquement séparé du réservoir et du moyen d'application.

Le moyen de chauffage est avantageusement sous la forme d'un dispositif de chauffage du type de ceux décrits dans les brevets US 6,009,884, US 5,853,010 ou bien encore US 6,220,252. Le dispositif de chauffage comprend avantageusement des éléments tels que par exemple un interrupteur permettant de mettre sous tension ou d'éteindre le dispositif, un voyant lumineux, dont l'état d'allumage ou le changement de couleur indique que le dispositif est à la température requise pour le prélèvement de la poudre, et notamment à une température suffisante pour provoquer la fusion de la composition.

Le dispositif de chauffage est avantageusement couplé ou intégré au moyen de prélèvement et/ou au moyen d'application, lequel moyen d'application comprend alors un moyen de préhension à l'intérieur duquel est disposée une batterie connectée à un fil chauffant, avantageusement en un alliage nickel/chrome, lui-même relié à une résistance qui chauffe la poudre de mascara prélevée.

On préfère que le moyen d'application intègre un moyen de chauffage. Un applicateur chauffant utilisable pour l'invention est par exemple décrit dans les demandes de brevet JP 2000-175725, JP 2003-310336. Un tel applicateur chauffant est commercialisé au Japon par la société MATSUSHITA sous la marque National® (modèle EH232).

Selon un mode de réalisation préféré, le dispositif de chauffage est agencé de manière à ne pas chauffer de manière sensible tout ou partie du moyen de préhension.

La quantité de mascara prélevé est mise en contact avec la partie chaude du moyen de prélèvement et/ou du moyen d'application.

Avantageusement, le mascara de l'invention est chauffé par le dispositif de chauffage avant ou pendant l'application du produit par l'utilisateur sur les fibres kératiniques, par exemple les cils.

Il est également possible de prévoir une recharge sous forme d'un réservoir dans lequel est conditionné le mascara de l'invention.

Un quatrième objet de l'invention vise un procédé de maquillage ou de traitement cosmétique des fibres kératiniques, ledit procédé comprenant une première étape de prélèvement de la poudre du mascara de l'invention, puis une étape de chauffage préalable ou concomitante à l'application dudit mascara, l'étape de chauffage visant à amener les particules de mascara au-delà de leur température de fusion, et enfin une étape d'application de la composition chauffée sur les fibres kératiniques, de façon à obtenir un film produisant un effet de maquillage et/ou de traitement cosmétique sur lesdites fibres.

L'invention vise en particulier un procédé de maquillage ou de soin cosmétique des fibres kératiniques, notamment des cils, dans lequel la poudre du mascara de l'invention est chauffée à une température supérieure ou égale à 35°C, et inférieure ou égale à 70°C.

Le procédé comprend notamment les étapes suivantes :
- prélèvement de la poudre du mascara selon l'invention, en quantité nécessaire pour réaliser au moins une application,
- chauffage de la quantité de poudre prélevée à une température suffisante pour obtenir la fusion des particules de la poudre,
- application du mascara ainsi chauffé sur les fibres kératiniques de façon à produire l'effet recherché.

### Description des figures

La figure 1 est une photo prise en microscopie électronique d'une fraction représentative de la poudre selon l'exemple 1, obtenue après broyage.
La figure 2 est une photo prise en microscopie électronique d'une fraction représentative de la poudre selon l'exemple 2, obtenue après broyage.

L'invention est illustrée par les exemples qui suivent. Dans ces exemples, les pourcentages sont massiques. Les noms des ingrédients correspondent à leur dénomination INCI.

### Exemple 1 : Mascara en poudre

On a préparé le mascara de composition suivante selon le procédé décrit ci-après.
75% PEG-32 (Pluracare® E1500 fourni par la société BASF, poids moléculaire 1400-1600 g/mol)
5% Polyvinylpyrolidone (PVP K30® solide 100% fourni par la société ISP, poids moléculaire 60 000 g/mol)
20% Oxydes de fer noir

On a chauffé à une température de 80°C toutes les matières premières de la formule excepté les pigments.

Après mélange sous agitation 1500 tours/min à l'aide d'un mélangeur adapté, on a ajouté les pigments puis on a laissé refroidir en maintenant l'agitation.

La masse obtenue après refroidissement a ensuite été broyée dans un broyeur à couteaux ou à broches de marque Alpine, de façon à réduire et homogénéiser la taille des particules du mascara.

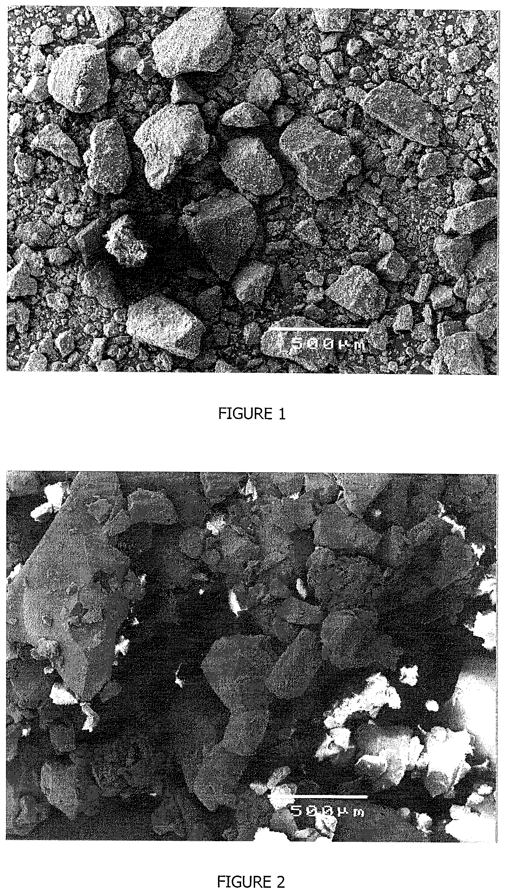

La granulométrie de la poudre obtenue (fig. 1) est constituée d'une population de particules dont une fraction a une granulométrie de quelques centaines de microns et une fraction de particules fines dont la taille est d'un ordre de grandeur allant du micron à quelques dizaines de microns.

### Mesure du point de fusion du mascara

Le point de fusion du mascara a été mesuré à l'aide d'un calorimètre à balayage différentiel (D.S.C.) de modèle STAR SW 9.30 de la société METTLER. Un échantillon de 12 mg de produit a été déposé dans un creuset est soumis à une première montée en température allant de -50°C à 100°C, à la vitesse de chauffe de 10°C/minute, laissé à la température de 100°C pendant 1 minute, puis a été refroidi de 100°C à -50°C à une vitesse de refroidissement de 5°C/minute, laissé à la température de -50°C pendant 4 minutes, et enfin soumis à une deuxième montée en température allant -50°C à 100°C, à la vitesse de chauffe de 10°C/minute. Pendant la deuxième montée en température, on a mesuré la variation de la différence de chaleur absorbée par le creuset vide et par le creuset contenant l'échantillon en fonction de la température. Le point de fusion du mascara est la valeur de la température correspondant au sommet du pic de la courbe représentant la variation de la différence de chaleur absorbée en fonction de la température.
Le point de fusion du mascara mesuré dans ces conditions était égal à 50°C.

### Exemple 2 : Mascara en poudre

On a préparé le mascara de composition suivante selon le même procédé que celui décrit à l'exemple 1.
70% copolymère VP/eicosène (Ganex® 220VF fourni par la société ISP, poids moléculaire 17 000 g/mol)
10% Polybutène (poids moléculaire moyen 2000 g/mol)
20% Oxydes de fer noirs

La granulométrie de la poudre obtenue (fig. 2) est constituée d'une population de particules dont la granulométrie est plus importante que celle obtenue à l'exemple 1, allant pour le présent exemple de quelques dizaines de microns jusqu'au millimètre.

Dans les mêmes conditions de broyages, la disparité des granulométries montre que la formule, notamment la composition en polymère, influence le comportement de la poudre vis-à-vis de cette étape.

Le point de fusion du mascara mesuré dans les mêmes conditions que celles de l'exemple 1 était égal à 44,8°C.

### Exemple 3 : Mascara en poudre

On a préparé le mascara de composition suivante selon le même procédé que celui décrit à l'exemple 1.
65% copolymère VP/eicosène (Ganex® 220VF fourni par la société ISP, poids moléculaire 17 000 g/mol)
15% Polybutène (poids moléculaire moyen 2000 g/mol)
20% Dispersion de pigments dans une cire*
**Dispersion de particules d'oxyde de Fer noir dans une cire (INCI=Hydrogenated Vegetable Oil) selon un ratio 50*/*50 p*/*p.*

Le point de fusion du mascara mesuré dans les mêmes conditions que celles de l'exemple 1 était égal à 41,1°C.

## Revendications

1. Mascara destiné à être chauffé pour être appliqué sur les fibres kératiniques, en particulier les cils, se présentant sous forme d'une poudre **caractérisée en ce que** la poudre est constituée de particules dont le diamètre moyen est compris entre 20 µm et 1 mm, et **en ce que** le mascara comprend au moins un premier polymère, de préférence filmogène, dont la température de fusion est comprise entre 35°C et 70°C, et un deuxième polymère, de préférence filmogène, dont la température de fusion est comprise entre 80 et 150°C, et **en ce que** le premier polymère est choisi parmi les copolymères de vinylpyrrolidone (VP) et d'alcène et le deuxième polymère est choisi parmi les polybutènes, ou **en ce que** le premier polymère est choisi parmi les polyéthylènes glycols et le deuxième polymère est choisi parmi les polyvinylpyrrolidones.

2. Mascara selon la revendication 1, **caractérisé en ce qu'**il est anhydre.

3. Mascara selon l'une des revendications précédentes, **caractérisé en ce qu'**il possède une température de fusion comprise entre 35 et 70°C, de préférence allant de 40 à 50°C.

4. Mascara selon l'une des revendications 1 à 3, **caractérisé en ce que** la proportion massique entre le premier polymère et le deuxième polymère est comprise entre 1 et 20, de préférence comprise entre 3 et 16.

5. Mascara selon l'une des revendications 1 à 4, **caractérisé en ce que** la quantité totale du premier polymère et du deuxième polymère est comprise entre 40 et 95% en poids, de préférence de 70 à 85% en poids, du poids total du mascara.

6. Mascara selon l'une des revendications 1 à 4, **caractérisé en ce que** le premier polymère est choisi parmi les copolymères VP/eicosène, VP/hexadécène, VP/triacontène, VP/styrène, et le deuxième polymère est choisi parmi les polybutènes de poids moléculaire moyen compris entre 300 et 2500 g/mol.

7. Mascara selon l'une des revendications 1 à 4, **caractérisé en ce que** le premier polymère est choisi parmi les polyéthylènes glycols de poids 23 août 2016
moléculaire moyen compris entre 1000 et 3000 g/mol ; et le deuxième polymère est choisi parmi les polyvinylpyrrolidones de poids moléculaire moyen compris entre 10 000 et 100 000 g/mol.

8. Mascara selon l'une des revendications précédentes, **caractérisé en ce qu'**il comprend de 1 à 25 % en poids, de préférence de 5% à 10 % en poids, d'au moins une cire par rapport au poids total du mascara.

9. Mascara selon l'une des revendications précédentes, **caractérisé en ce qu'**il comprend de 5% à 30 % en poids, de préférence de 10 à 25% en poids, de matière colorante par rapport au poids total du mascara.

10. Mascara selon l'une des revendications précédentes, **caractérisé en ce qu'**il comprend moins de 5% en poids d'un composé liquide à température ambiante, tel qu'une huile ou un solvant volatile.

11. Procédé de fabrication du mascara selon l'une des revendications 1 à 10, comprenant :
- une étape de mélange à chaud des composants du mascara de façon à former une phase liquide,
- une étape de refroidissement,
- puis une étape visant à diviser la masse préparée précédemment de façon à obtenir une poudre formée de particules solides.

12. Procédé selon la revendication 11, **caractérisé en ce que** l'étape de division est réalisée par broyage de ladite phase liquide qui a été solidifiée par refroidissement.

13. Procédé selon la revendication 11, **caractérisé en ce que** l'étape de division est réalisée par atomisation de ladite phase liquide.

14. Kit de maquillage comprenant un mascara selon l'une quelconque des revendications 1 à 10 conditionné dans un réservoir, ainsi que des moyens de prélèvement, d'application et de chauffage dudit mascara.

15. Kit selon la revendication 14, **caractérisé en ce que** le moyen de prélèvement est couplé ou intégré au réservoir.

16. Kit selon les revendications 14 ou 15, **caractérisé en ce que** le réservoir et/ou le moyen de prélèvement comprennent des moyens de distribution de la poudre du mascara.

17. Kit selon l'une quelconque des revendications 14 à 16, **caractérisé en ce que** le moyen de chauffage est couplé ou intégré au moyen de prélèvement et/ou au moyen d'application.

18. Procédé de maquillage ou de soin cosmétique des fibres kératiniques, notamment des cils, comprenant les étapes suivantes :
- prélèvement de la poudre du mascara selon l'une des revendications 1 à 10, en quantité nécessaire pour réaliser au moins une application,
- chauffage de la quantité de poudre prélevée à une température suffisante pour obtenir la fusion de la poudre,
- application du mascara ainsi chauffé sur les fibres kératiniques de façon à produire l'effet recherché.

19. Procédé selon la revendication 18, **caractérisée en ce que** le chauffage est réalisé à une température comprise entre 35 et 70°C.

## Patentansprüche

1. Mascara, dazu bestimmt, erwärmt zu werden, um auf Keratinfasern aufgebracht zu werden, insbesondere auf die Wimpern, und welche in Form eines Puders vorliegt, **dadurch gekennzeichnet, dass** das Puder aus Teilchen besteht, deren mittlerer Durchmesser zwischen 20 µm und 1 mm liegt, und dadurch, dass die Mascara mindestens ein erstes, bevorzugt filmbildendes, Polymer, dessen Schmelztemperatur zwischen 35 °C und 70 °C liegt, und ein zweites, bevorzugt filmbildendes, Polymer, dessen Schmelztemperatur zwischen 80 und 150 °C liegt, umfasst, und dadurch, dass das erste Polymer aus Vinylpyrrolidon- (VP-) und Alken-Copolymeren ausgewählt ist, und das zweite Polymer aus Polybutenen ausgewählt ist, oder dadurch, dass das erste Polymer aus Polyethylenglykolen ausgewählt ist, und das zweite Polymer aus Polyvinylpyrrolidonen ausgewählt ist.

2. Mascara gemäß Anspruch 1, **dadurch gekennzeichnet, dass** diese wasserfrei ist.

3. Mascara gemäß Anspruch 1, **dadurch gekennzeichnet, dass** diese eine Schmelztemperatur zwischen 35 und 70 °C, bevorzugt von 40 bis 50 °C, aufweist.

4. Mascara gemäß einem der Ansprüche 1 bis 3, **dadurch gekennzeichnet, dass** das Massenverhältnis zwischen dem ersten Polymer und dem zweiten Polymer zwischen 1 und 20, bevorzugt zwischen 3 und 16, liegt.

5. Mascara gemäß einem der Ansprüche 1 bis 4, **dadurch gekennzeichnet, dass** die Gesamtmenge des ersten Polymers und des zweiten Polymers zwischen 40 und 95 Gew.-%, bevorzugt von 70 bis 85 Gew.-%, bezogen auf das Gesamtgewicht der Mascara, beträgt.

6. Mascara gemäß einem der Ansprüche 1 bis 4, **dadurch gekennzeichnet, dass** das erste Polymer aus VP/Eicosen-, VP/Hexadecen-, VP/Triaconten-, VP/Styrol-Copolymeren ausgewählt ist, und das zweite Polymer aus Polybutenen mit einem mittleren Molekulargewicht zwischen 300 und 2500 g/mol ausgewählt ist.

7. Mascara gemäß einem der Ansprüche 1 bis 4, **dadurch gekennzeichnet, dass** das erste Polymer aus Polyethylenglykolen mit einem mittleren Molekulargewicht zwischen 1000 und 3000 g/mol ausgewählt ist und das zweite Polymer aus Polyvinylpyrrolidonen mit einem mittleren Molekulargewicht zwischen 10 000 und 100 000 g/mol ausgewählt ist.

8. Mascara gemäß einem der vorhergehenden Ansprüche, **dadurch gekennzeichnet, dass** diese von 1 bis 25 Gew.-%, bevorzugt von 5 Gew.-% bis 10 Gew.-%, mindestens eines Wachses im Verhältnis zum Gesamtgewicht der Mascara umfasst.

9. Mascara gemäß einem der vorhergehenden Ansprüche, **dadurch gekennzeichnet, dass** diese von 5 Gew.-% bis 30 Gew.-%, bevorzugt von 10 bis 25 Gew.-%, eines Farbstoffs im Verhältnis zum Gesamtgewicht der Mascara umfasst.

10. Mascara gemäß einem der vorhergehenden Ansprüche, **dadurch gekennzeichnet, dass** diese weniger als 5 Gew.-% einer Verbindung umfasst, die bei Umgebungstemperatur flüssig ist, wie ein Öl oder ein flüchtiges Lösungsmittel.

11. Verfahren zur Herstellung einer Mascara gemäß einem der Ansprüche 1 bis 10, umfassend:
- einen Schritt des Heißmischens der Bestandteile der Mascara, um eine Flüssigphase zu bilden,
- einen Schritt des Abkühlens,
- dann einen Schritt, der darauf gerichtet ist, die zuvor hergestellte Masse zu teilen, um ein aus festen Teilchen gebildetes Puder zu erhalten.

12. Verfahren gemäß Anspruch 11, **dadurch gekennzeichnet, dass** der Schritt des Teilens durch Zerkleinern der Flüssigphase durchgeführt wird, die durch Abkühlen verfestigt wurde.

13. Verfahren gemäß Anspruch 11, **dadurch gekennzeichnet, dass** der Schritt des Teilens durch Zerstäuben der Flüssigphase durchgeführt wird.

14. Make-up-Kit, umfassend eine Mascara gemäß einem der Ansprüche 1 bis 10, die in einem Behälter verpackt ist, sowie Mittel zum Entnehmen, Aufbringen und Erwärmen der Mascara.

15. Kit gemäß Anspruch 14, **dadurch gekennzeichnet, dass** das Mittel zum Entnehmen mit dem Behälter gekoppelt oder integriert ist.

16. Kit gemäß den Ansprüchen 14 oder 15, **dadurch gekennzeichnet, dass** der Behälter und/oder das Mittel zum Entnehmen Mittel zum Verteilen des Pulvers der Mascara umfassen.

17. Kit gemäß einem der Ansprüche 14 bis 16, **dadurch gekennzeichnet, dass** das Mittel zum Erwärmen mit dem Mittel zum Entnehmen und/oder mit dem Mittel zum Aufbringen gekoppelt oder integriert ist.

18. Verfahren zum Schminken oder zur kosmetischen Pflege von Keratinfasern, insbesondere den Wimpern, umfassend die folgenden Schritte:
- Entnehmen des Puders der Mascara gemäß einem der Ansprüche 1 bis 10 in einer Menge, die zum Vornehmen mindestens eines Aufbringens notwendig ist,
- Erwärmen der entnommenen Pudermenge auf eine ausreichende Temperatur, um das Schmelzen des Puders zu erhalten,
- Aufbringen der so erwärmten Mascara auf die Kertainfasern, um den gewünschten Effekt zu erzielen.

19. Verfahren gemäß Anspruch 18, **dadurch gekennzeichnet, dass** das Erwärmen auf eine Temperatur zwischen 35 und 70 °C durchgeführt wird.

## Claims

1. A mascara intended to be heated in order to be applied on keratin fibers, in particular the eyelashes, said mascara being in the form of a powder, wherein said powder consists of particles having a mean diameter between 20 µm and 1 mm, wherein the mascara comprises at least a first polymer, preferably a film-forming polymer, whose melting point is between 35°C and 70°C, and a second polymer, preferably a film-forming polymer, with a melting point of between 80°C and 150°C, and wherein the first polymer is chosen from copolymers of vinylpyrrolidone (VP) and alkene, and the second polymer is chosen from polybutenes, or wherein the first polymer is chosen from polyethylene glycols, and the second polymer is chosen from polyvinylpyrrolidones.

2. The mascara of claim 1, wherein said mascara is anhydrous.

3. The mascara as claimed in one of preceding claims, whose melting point is between 40°C and 50°C.

4. The mascara as claimed in one of claims 1 to 3, wherein the mass proportion between the first polymer and the second polymer is between 1 and 20 and preferably between 3 and 16.

5. The mascara as claimed in one of claims 1 to 4, wherein the total amount of the first polymer and of the second polymer is between 40% and 95% by weight and preferably from 70% to 85% by weight relative to the total weight of the mascara.

6. The mascara as claimed in one of claims 1 to 4, wherein the first polymer is chosen from VP/eicosene, VP/hexadecene, VP/triacontene and VP/styrene copolymers, and the second polymer is chosen from polybutenes with an average molecular weight of between 300 and 2500 g/mol.

7. The mascara as claimed in one of claims 1 to 4, wherein the first polymer is chosen from polyethylene glycols with an average molecular weight of between 1000 and 3000 g/mol; and the second polymer is chosen from polyvinylpyrrolidones with an average molecular weight of between 10 000 and 100 000 g/mol.

8. The mascara of anyone of the preceding claims, wherein said mascara comprises from 1% to 25% by weight and preferably from 5% to 10% by weight of at least one wax relative to the total weight of the mascara.

9. The mascara of anyone of the preceding claims, wherein said mascara comprises from 5% to 30% by weight and preferably from 10% to 25% by weight of dyestuff relative to the total weight of the mascara.

10. The mascara of anyone of the preceding claims, wherein said mascara comprises less than 5% by weight of a compound that is liquid at room temperature, such as an oil or a volatile solvent.

11. A process for manufacturing the mascara as claimed in one of claims 1 to 10, comprising:
- a step of hot-mixing of the mascara components, so as to form a liquid phase,
- a step of cooling,
- and then a step for dividing the mass prepared previously so as to obtain a powder formed from solid particles.

12. The process of claim 11, wherein the dividing step is performed by milling said liquid phase which has been solidified on cooling.

13. The process of claim 11, wherein the dividing step is performed by atomization of said liquid phase.

14. A makeup kit comprising a mascara as claimed in any one of claims 1 to 10, conditioned in a reservoir, and also means for taking up, applying and heating said mascara.

15. The kit of claim 14, wherein the take-up means is coupled to or integrated into the reservoir.

16. The kit of claim 14 or 15, wherein the reservoir and/or the take-up means comprise means for dispensing the mascara powder.

17. The kit as claimed in any one of claims 14 to 16 wherein the heating means is coupled to or integrated into the take-up means and/or the application means.

18. A process for making up or for the cosmetic care of keratin fibers, especially the eyelashes, comprising the following steps:
- taking up the mascara powder as claimed in one of claims 1 to 10, in an amount necessary to make at least one application,
- heating the amount of powder taken up to a temperature sufficient to obtain melting of the powder,
- applying the mascara thus heated to the keratin fibers so as to produce the desired effect.

19. The process of claim 18, wherein the heating is performed at a temperature of between 35 and 70°C.
